# EUROPEAN PATENT APPLICATION

(11) **EP 2 005 964 A2**
(43) Date of publication of application: **24.12.2008**
(21) Application number: 06753035.2
(22) Date of filing: 26.06.2006
(51) Int. Cl.: A61K 33/24, A61P 3/12

(54) **THE MEDICAMENT FOR TREATING HYPERPHOSPHEREMIA AND PREPARATION THEREOF**

(30) Priority: 24.03.2006 CN 200610043267
(71) Applicant: LI, Shibiao, Shandong 250013 (CN); Qiao, Min, Shandong 250013 (CN); Zhang, Weisheng, Shandong 250013 (CN)
(72) Inventor: LI, Shibiao, Shandong 250013 (CN); Qiao, Min, Shandong 250013 (CN); Zhang, Weisheng, Shandong 250013 (CN)
(74) Representative: Pereira Toña, Maria Irache
(86) International application number: PCT/CN2006/001463
(87) International publication number: WO 2007/109929

(57) **Abstract**

The medicament for treating hyperphospheremia and preparative method thereof are provided. The medicament comprises lanthanum polystyrene sulfonate as the active ingredient and pharmaceutic adjuvant. The preparative method in the invention comprises: acidizing polystyrene sulfonate by hydrochloric acid, and soaking or eluting the obtained polystyrene sulfonic acid by water-solubility lanthanum salt solution, then washing to neutral PH by water, washing out non-exchanged redundant lanthanum ions, drying the obtained lanthanum polystyrene sulfonate and crushing to powders, the lanthanum in the lanthanum polystyrene sulfonate is 14-22 wt, and the said lanthanum polystyrene sulfonate and usual dose pharmaceutic adjuvant are prepared to the medicament suitable for gastrointestinal tract according to common preparative method. The medicament in the invention can be administered by gastrointestinal tract route, and form insolubility conjugate with phosphate in the digestive tract, then eliminate from digestive tract, thus achieve the purpose of treating hyperphospheremia.

## Description

### Technique information:

This invention relates to medicines, particularly for a medicine used for curing hyperphosphatemia and its preparation method.

### Background Techniques:

Hyperphosphatemia is the complication of renal failure, hypoparathyroidism, and some other diseases; it could result in serious disorders of calcium and phosphorous metabolism.

Nowadays, hyperphosphatemia is generally treated with dialysis, or oral intake of aluminum salts, calcium salts, or lanthanum carbonate hydrates. However, they all have some negative effects on human body: dialysis could cause considerable harm to human body, and it could not achieve much effects in decreasing the concentration of phosphates; oral intake of aluminum salts and calcium salts has considerable toxic and side effects by causing damage to patient's kidneys, skin, inner organs, brain and bones, and for this reason oral intake of these salts are usually used under restriction in clinical practice; oral intake of lanthanum carbonate hydrates could only dissolve and remove the phosphates containing in the food in solutions of a pH value approximately equal to that of gastric juice, and it is insoluble in solutions at a high pH value approximately equal to that of intestinal juice.

Therefore, oral intake of lanthanum carbonate could not completely remove the phosphates in intestine. Apart from all these medicines, a patent in the US reported an amine polymer used for removing the phosphate ions out of patients' bodies; though it could effectively remove the phosphates in stomach and intestine, the high synthesizing cost and the accordingly expensive production cost impose heavy economic burden on patients. For this reason, all through the years our technicians in this area are endeavoring to find a medicine able to function in all solutions at different pH values all through the gastrointestinal tract. It should also be inexpensive for more patients to afford it.

### The contents of the invention:

The invention is aimed to provide a medicine used for the curing of hyperphosphatemia and its preparation method. The medicine is made of polystyrene sulfonic lanthanum, which is the effective ingredient of this medicine, and some pharmaceutical excipients. The medicine is given to the patient by gastrointestinal drug delivery to turn phosphates in the alimentary tract into insoluble conjugates, which are then discharged out of the body; thus hyperphosphatemia could be effectively treated.

To fulfill the purpose mentioned above, in this invention we adopted the following technical method: we made a medicine curing hyperphosphatemia composed of polystyrene sulfonic lanthanum, which is the effective ingredient of the medicine, and some other pharmaceutical excipients. The following is the general molecular formula of polystyrene sulfonic lanthanum: 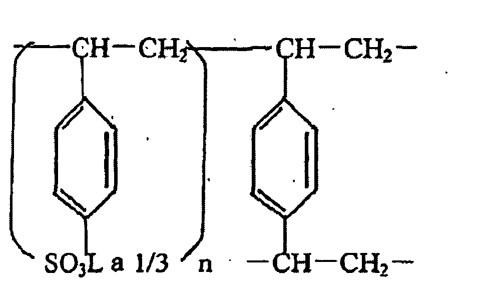 In the formula n refers to an integral number.

The mass of lanthanum in the polystyrene sulfonic lanthanum above mentioned is 14~22%.

The mass of lanthanum in the polystyrene sulfonic lanthanum above mentioned is 16~22%.

The medicine above is used for curing hyperphosphatemia. Polystyrene sulfonic lanthanum, the effective ingredient of the medicine, is applied in the preparation of medicines used for curing hyperphosphatemia through gastro- intestinal drug delivery.

The formulation of the medicine above mentioned can take various forms, including powder, capsules, tablets, dry suspension, suspension and granules.

The preparation method of the medicine curing hyperphosphatemia above mentioned is given as follows: polystyrene sulfonic salts is acidized with HCl to produce polystyrene sulfonate, which is then soaked or washed with soluble lanthanum salt solutions and rinsed rinsing with distilled water to a neutral pH value. After surplus lanthanum ions un-exchanged are removed, dry the obtained polystyrene sulfonic lanthanum and pulverize it into powder; in polystyrene sulfonic lanthanum the mass of lanthanum is 14~22%. Then polystyrene sulfonic lanthanum powder, in which the mass of lanthanum being 14∼22%, is mixed with conventional amount of pharmaceutical excipients to produce a gastrointestinal medicine with conventional preparation method.

After polystyrene sulfonic salts is acidized with HCl to produce polystyrene sulfonate, polystyrene sulfonate is soaked or washed with soluble lanthanum salt solutions at the temperature of 0°C - 60°C, and then laid aside or washed for 5 to 30 hours. In the polystyrene sulfonic lanthanum prepared with the method above mentioned the mass of the lanthanum is 14~22%. The medicine, prepared with the method above mentioned, which is made of polystyrene sulfonic lanthanum and pharmaceutical excipients, can take various formulation forms, including powder, capsules, tablets, dry suspension, suspension and granules. The soluble lanthanum salt solution above mentioned in the preparation method could be lanthanum nitrate solution, lanthanum chloride solution, lanthanum sulfate solution, or lanthanum acetate solution.

In clinical practice, the researchers of the invention discovered that polystyrene sulfonic lanthanum could not only effectively remove the phosphates in the whole alimentary tract, including the stomach, the small intestine and the large intestine, but also decrease the release of lanthanum from the polystyrene sulfonic lanthanum into the digestive juice when the phosphates in the alimentary tract decrease, and increase the release of lanthanum when the phosphates increase. In consideration of this, we drew the conclusion that polystyrene sulfonic lanthanum is of good clinical significance.

Further studies show that polystyrene sulfonic lanthanum could attain significant effects in removing phosphates in the stomach, the small intestine and the large intestine. Relevant experiment is given as follows:
**Priming solution 1:** dissolve 14g Na2HPO4 and 8.5g NaCl in 1000ml distilled water, and adjust the solution to the pH value of 3. Then filter the solution and quantify the phosphate concentration.
**Priming solution 2:** dissolve 14g Na2HPO4 and 8.5g NaCl in 1000ml distilled water, and adjust the solution to the pH value of 6.8. Then filter the solution and quantify the phosphate concentration.
**Priming solution 3:** dissolve 14g Na2HPO4 and 8.5g NaCl in 1000ml distilled water, and adjust the solution to the pH value of 7.8. Then filter the solution and quantify the phosphate concentration.
Add certain amounts of polystyrene sulfonic lanthanum into priming solution 1, priming solution 2, and priming 3 respectively and set the molar ratio of lanthanum to phosphates at 3:1. Chum up the priming solutions at 37°C, and collect samples at intervals to quantify the amount of phosphates and calculate the portion of the phosphate removed. The results are given in the following table:

| Time used (min) | The portion of the phosphate removed (%) | | |
|---|---|---|---|
| | priming solution 1 | priming solution2 | priming solution3 |
| 10 | 94.5 | 97.2 | 96.4 |
| 20 | 95.2 | 98.0 | 96.6 |
| 30 | 95.7 | 98.3 | 96.8 |
| 60 | > 96.0 | > 98.0 | > 98.0 |

From the table above we can see that polystyrene sulfonic lanthanum could attain basically similar effects in removing phosphates in solutions at pH values equal to either that of the stomach, that of the small intestine, or that of the large intestine.

In order to further verify the safety of this medicine, we performed the following experiment to demonstrate that polystyrene sulfonic lanthanum could decrease the release of lanthanum ions when the phosphates in the alimentary tract decrease:
Mix polystyrene sulfonic lanthanum and different amount of phosphates in 0.85% NaCl solutions at pH value of 6.8 and set the molar ratios of lanthanum to phosphate in different solutions at 1:0.2, 1:0.1, 1:0.05 and 1:0 respectively. Chum the solutions up for 10 minutes at 37°C. Then quantify the lanthanum concentration in the solution and calculate the amount of lanthanum still existing in polystyrene sulfonic lanthanum. The results are 19%, 43%, 68%, and 91% respectively for the different molar ratios. This could testify that the release of lanthanum decreases as the phosphates in the solutions decrease.

Add 0.5g lanthanum carbonate into 100ml 0.85% NaCl (pH=6.8) solution and 100ml 0.85% NaCl (pH=7.8) solution respectively, and then add proper amount of Na2HPO4 into the solutions. Chum the solutions at 37°C, and take samples to filter and quantify the concentration of phosphates at intervals (by sampling at 10 min, 30 min, 40 min, and 60 min after the churning). The portion of the phosphate removed is calculated in the following table:

| Time | **Portion of phosphates removed (%) in 0.85% NaCl pH6.8 solution** | **Portion of phosphates removed (%) in 0.85% NaCl Ph7.8solution** |
|---|---|---|
| 10 | 6.7 | 1.4 |
| 30 | 13.7 | < 2.0 |
| 40 | 16.9 | < 2.0 |
| 60 | 20.8 | < 2.0 |

The above results show that lanthanum carbonate removes little amount of phosphates in solutions at pH value near that of the small intestine, and it hardly remove any phosphates in solutions at pH value near that of the large intestine.

The mass of lanthanum in polystyrene sulfonic lanthanum is determined by many factors in the preparation method given above, including the reaction temperature and time of soluble lanthanum salts. When the soluble lanthanum salts are of a certain concentration, then the longer the time is, the more the lanthanum will be found in polystyrene sulfonic lanthanum, and the higher the temperature is, the more lanthanum will be. The data in the following table further testify the above discovery.

In the following table, Sample 1, 2, 3 and 4 are polystyrene sulfonic lanthanum samples obtained under different conditions with the preparation method given in this invention. In these samples the mass ratio of polystyrene sulfonate to lanthanum nitrate is 1:2.

| Sample | Temperature (°C) | Time (Hr) | Mass of lanthanum in poly-Styrene sulfonic lanthanum (%) |
|---|---|---|---|
| 1 | 50 | 12 | 20 |
| 2 | 50 | 8 | 18 |
| 3 | 30 | 8 | 16 |
| 4 | 20 | 16 | 20 |

In the following table, Sample 1, 2, 3 and 4 are polystyrene sulfonic lanthanum samples obtained under different conditions with the preparation method given in this invention. In these samples the mass ratio of polystyrene sulfonic salts to lanthanum chloride is 1:2.

| Sample | Temperature (°C) | Time (Hr) | Mass of lanthanum in poly-Styrene sulfonic lanthanum (%) |
|---|---|---|---|
| 1 | 50 | 16 | 22 |
| 2 | 50 | 8 | 20 |
| 3 | 30 | 12 | 18 |
| 4 | 20 | 8 | 17 |

The temperature for the above table could be anywhere between 0 °C and 60°C, but a lower temperature will require a longer time to react. 30-50°C is optimal for industrial production.

The following is the pharmacodynamic experiment of the invention:
To demonstrate the curing effect of polystyrene sulfonic lanthanum on CRF hyperphosphatémie, we performed the following experiment:

The test medicine: polystyrene sulfonic lanthanum.

Experiment animal: 30 Wistar male rats weighting 180~220g; divide the rats at random into: (1) a normal control group of 10 rats, which are fed with forage and given gastric lavage after two weeks with 2ml water per 200g body weight every day, (2) a model control group of 10 rats which are given gastric lavage with 2ml 0.2% adenine solution per 200g body weight every day, and (3) a therapy group of 10, which are first given gastric lavage with adenine solution for two weeks and then with polystyrene sulfonic lanthanum 200mg per 1000g body weight every day. After giving the rats gastric lavage for 6 weeks, we collect blood in the 6^{th} week and quantify the serum phosphorous (P). The results are given in the table below:

| Group | Number | P(mmol/L) in the 3^{rd} week | P(mmol/L) in the 6^{th} week |
|---|---|---|---|
| Normal group | 10 | 2.84±0.21 | 2.97±0.28 |
| Pathological group | 10 | 4.10±0.27 | 4.58±0.34 |
| Therapy group | 10 | 3.22±0.31 | 3.14±0.2 |

Comparing the pathological group with the normal group, P<0.05; comparing the therapy group and the pathological group, P<0.05.

**Conclusion:** polystyrene sulfonic lanthanum could decrease the serum phosphorous of rats with hyperphosphatémie.

All the results of the above experiments show that polystyrene sulfonic lanthanum could effectively remove the phosphate in stomach, small intestine and large intestine by turning the phosphates in the alimentary tract into insoluble conjugates which are then discharged out of the body. Then hyperphosphatemia is treated. Besides, polystyrene sulfonic phosphate imposes little side effect on human body, for the release of lanthanum ions from polystyrene sulfonic phosphate would decrease when the phosphates in the alimentary tract decrease, and polystyrene sulfonic lanthanum in human body could dissociate into polystyrene sulfonate, which cannot be absorbed by human body. In patent document 96193918.4 of China has published the experiment proving that polystyrene sulfonic lanthanum needs no lanthanum to bind phosphates; this provides a further convincing evidence of the non-toxicity of polystyrene sulfonic lanthanum.

It costs not much to produce powder, granules, capsules, dry suspension, suspension and tablets above mentioned in the invention, which are made of polystyrene sulfonic lanthanum and the pharmaceutical excipients with the preparation method above mentioned. To attain similar effects in removing the phosphates in the gastrointestinal tract of patients, it is less expensive than the production cost of amine polymers; thus more people could afford it now. Since this could cut down the expense of patients who need to take long term medication, it provides more patients with a medicine which not only has low side effects but also could remove the phosphates in the gastrointestinal tract effectively.

### Implementation method:

The medicine curing hyperphosphatemia above mentioned in the invention is a gastrointestinal medicine composed of polystyrene sulfonic lanthanum, which is the effective ingredient, and pharmaceutical excipients. The structural formula of polystyrene sulfonic lanthanum is: 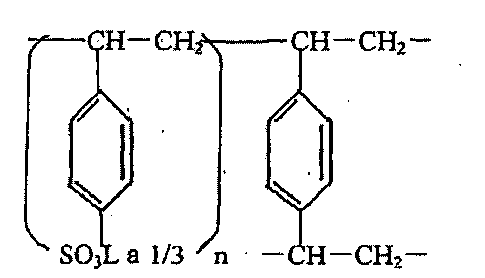 In the formula n refers to an integral number.

The mass of lanthanum in polystyrene sulfonic lanthanum is generally 14-22%; if it is lower than 14%, more medicine must be taken by the patients. This invention can be further optimized by increasing the mass of lanthanum in polystyrene sulfonic lanthanum to 16~22%. The formulation of the invention could be powder, granules, capsules, dry suspension, suspension or tablets. Polystyrene sulfonic lanthanum powder, with lanthanum constituting 16%, 17%, 18%, 19%, 20%, 21% or 22% of the total mass, could be mixed up with conventional amounts of pharmaceutical excipients and Aspartame to produce powder for gastric lavage (the powder can also be made without adding in pharmaceutical excipients). Hydroxypropyl methyl cellulose could be a substitution for pharmaceutical excipients; it could be mixed up with polystyrene sulfonic lanthanum to produce dry suspension; if water is added in, then we can get suspension. With conventional capsule excipients or tablet excipients equal to the amount of conventional pharmaceutical excipients we can produce capsules and tablets. The pharmaceutical excipients applied could be any sort used in medical practice.

The medicine made of polystyrene sulfonic lanthanum above mentioned in the invention could cure any symptom caused by hyperphosphatemia by gastrointestinal drug intake. The experiment on the invention shows that polystyrene sulfonic lanthanum could be applied in any medicine prepared for curing hyperphosphatemia by gastrointestinal drug intake. Oral intake of the invention should be 3~10g polystyrene sulfonic lanthanum for adults, and children or patients of special body constitution should follow the doctor's advice.

The medicine above mentioned used for curing hyperphosphatemia could be prepared with the following procedures: First polystyrene sulfonic salts are acidized with HCl to turn into polystyrene sulfonate, which is then soaked or washed with soluble lanthanum salt solution. Return the solution to a neutral pH by rinsing it with distilled water, and remove all the un-exchanged lanthanum ions. Dry the obtained polystyrene sulfonic lanthanum and pulverize it into powder; in polystyrene sulfonic lanthanum the mass of lanthanum is 14-22%. Polystyrene sulfonic lanthanum powder in which the mass of lanthanum is 14-22% is considered as the effective ingredient, which are mixed with conventional amount of pharmaceutical excipients to produce medicine with conventional preparation method.

The polystyrene sulfonic salts above mentioned could be polystyrene sulfonic sodium, polystyrene sulfonic calcium, polystyrene sulfonic potassium, polystyrene sulfonic magnesium, and etc. Among them we can choose polystyrene sulfonic sodium or polystyrene sulfonic calcium to cut down the production cost. The soluble lanthanum salt solution could be lanthanum nitrate solution, lanthanum chloride solution, lanthanum sulfate solution or lanthanum acetate solution. The polystyrene sulfonic salts above mentioned are acidized with HCl to turn into polystyrene sulfonate, which is then soaked or washed with soluble lanthanum solution at 0~60 °C (optimized at 20°C to 50°C), and laid aside or washed for 4-35 hours (optimized for 8-60 hours). Considering that the time of laying aside or washing is related to the concentration of the soluble lanthanum solution, we can adjust the time in light of the concentration. In a word, we have to wait until the reaction is complete.

To further refine the medicine, we can first soak and rinse the polystyrene sulfonic salts with distilled water, and then remove the impurities by soaking it in ethanol. Of course this procedure could be arranged after the polystyrene sulfonic lanthanum is obtained, but it may cause inconvenience in production. The concentration of the soluble lanthanum solution could be made as required by adjusting the time of laying aside or washing. In polystyrene sulfonic lanthanum obtained with the above method, the mass of lanthanum could be restricted within the range of 14%-22% (optimized for 16%-22%). Distilled water is the best choice for the water in preparation.

The full name of the polystyrene sulfonic salt mentioned in the invention is polystyrene sulfonic salt positive ion exchange resin.

Here are some examples of preparation:
1. 100g polystyrene sulfonic sodium is acidized with HCl to turn into polystyrene sulfonate, which is then soaked or washed with 400ml 50% lanthanum nitrate solution at 50°C for 8 hrs. Return the solution to a neutral pH by rinsing it with distilled water, and remove all the un-exchanged lanthanum ions. Dry the obtained polystyrene sulfonic lanthanum and pulverize it into powder; in polystyrene sulfonic lanthanum the mass of lanthanum is 16%. Then polystyrene sulfonic lanthanum powder is mixed with conventional amount of pharmaceutical excipients to produce medicine with conventional preparation method.
2. 100g polystyrene sulfonic calcium is acidized with HCl to turn into polystyrene sulfonate, which is then soaked or washed with 500ml 40% lanthanum nitrate solution at 20°C for 20 hrs. Return the solution to a neutral pH by rinsing it with distilled water, and remove all the un-exchanged lanthanum ions. Dry the obtained polystyrene sulfonic lanthanum and pulverize it into powder; in polystyrene sulfonic lanthanum the mass of lanthanum is 14%. Then polystyrene sulfonic lanthanum powder is mixed with conventional amount of pharmaceutical excipients to produce medicine with conventional preparation method.
3. 100g polystyrene sulfonic sodium is acidized with HCl to turn into polystyrene sulfonate, which is then packed with wet method and washed with 400ml 50% lanthanum chloride solution while maintaining a temperature of 60°C in the cylinder for 4 hrs. It could be washed again, or even for a third time. Return the solution to a neutral pH by rinsing it with distilled water, and remove all the un-exchanged lanthanum ions. Dry the obtained polystyrene sulfonic lanthanum and pulverize it into powder; in polystyrene sulfonic lanthanum the mass of lanthanum is 19%. Then polystyrene sulfonic lanthanum powder is mixed with conventional amount of pharmaceutical excipients to produce medicine with conventional preparation method.
4. First soak and rinse 100g polystyrene sulfonic sodium in distilled water until the rinsing water becomes clear, and remove the impurities by soaking it in ethanol. After that, acidize it with HCl to turn it into polystyrene sulfonate, which is then soaked with 400ml 50% lanthanum nitrate solution at 30°C for 8 hrs. Return the solution to a neutral pH by rinsing it with distilled water, and remove all the un-exchanged lanthanum ions. Dry the obtained polystyrene sulfonic lanthanum and pulverize it into powder; in polystyrene sulfonic lanthanum the mass of lanthanum is 16%. Then in the polystyrene sulfonic lanthanum powder obtained we can add 1 % Aspartame to produce powder, apply light syrup as the adhesive to produce granules, add 2.5% hydroxypropyl methyl cellulose and mix them up to produce dry suspension, which could be further made into suspension by adding water in, or add conventional amounts of pharmaceutical excipients to produce capsules or tablets with conventional preparation method.
5. First soak and rinse 100g polystyrene sulfonic sodium in distilled water until the rinsing water becomes clear, and remove the impurities by soaking it in ethanol. After that, acidize it with HCl to turn it into polystyrene sulfonate, which is then packed with wet method and washed with 400ml 50% lanthanum chloride solution while maintaining a temperature of 50°C in the cylinder for 16 hrs. It could be washed again, or even for a third time. Return the solution to a neutral pH by rinsing it with distilled water, and remove all the un-exchanged lanthanum ions. Dry the obtained polystyrene sulfonic lanthanum and pulverize it into powder; in polystyrene sulfonic lanthanum the mass of lanthanum is 22%. Then in the polystyrene sulfonic lanthanum powder obtained we can add 5% starch paste to produce tablets.
6. First soak and rinse 100g polystyrene sulfonic calcium in distilled water until the rinsing water becomes clear, and remove the impurities by soaking it in ethanol. After that, acidize it with HCl to turn it into polystyrene sulfonate, which is then soaked with 500ml 40% lanthanum sulfate solution at 20°C for 8 hrs. Return the solution to a neutral pH by rinsing it with distilled water, and remove all the un-exchanged lanthanum ions. Dry the obtained polystyrene sulfonic lanthanum and pulverize it into powder below 80°C; in polystyrene sulfonic lanthanum the mass of lanthanum is 14%. Then in the polystyrene sulfonic lanthanum powder obtained we can add conventional amounts of pharmaceutical excipients to produce medicine with conventional preparation method.
7. First soak and rinse 100g polystyrene sulfonic magnesium in distilled water until the rinsing water becomes clear, and remove the impurities by soaking it in ethanol for over 8 hrs. After that, acidize it with HCl to turn it into polystyrene sulfonate, which is then soaked with 600ml 30% lanthanum sulfate solution at 35°C for three times, and laid aside for 8 hrs.
   For the first two times, return the solution to a neutral pH by rinsing it with distilled water, and for the third time remove all the un-exchanged lanthanum ions by rinsing it with distilled water. Dry the obtained polystyrene sulfonic lanthanum and pulverize it into powder; in polystyrene sulfonic lanthanum the mass of lanthanum is 18%. Then in the polystyrene sulfonic lanthanum powder obtained we can add conventional amounts of pharmaceutical excipients to produce medicine with conventional preparation method.
8. First soak and rinse 100g polystyrene sulfonic potassium in distilled water until the rinsing water becomes clear, and remove the impurities by soaking it in ethanol for over 8 hrs. After that, acidize it with HCl to turn it into polystyrene sulfonate, which is then soaked with 400ml 50% lanthanum sulfate solution twice, and then lay it aside at 20°C for 16 hrs.

For the first time, return the solution to a neutral pH by rinsing it with distilled water, and for the second time remove all the un-exchanged lanthanum ions by rinsing it with distilled water. Dry the obtained polystyrene sulfonic lanthanum and pulverize it into powder; in polystyrene sulfonic lanthanum the mass of lanthanum is 20%. Then in the polystyrene sulfonic lanthanum powder obtained we can add conventional amounts of pharmaceutical excipients to produce medicine with conventional preparation method.

In polystyrene sulfonic lanthanum above mentioned in the invention, the mass of lanthanum is 14-22%; it is determined by defining the total mass of polystyrene sulfonic lanthanum as 100%.

A further interpretation of the mass of lanthanum in polystyrene sulfonic lanthanum is as follows: it's the portion the mass of lanthanum takes in the polystyrene sulfonate matrix, and the matrix doesn't include lanthanum. For example: the saying "in polystyrene sulfonic lanthanum above mentioned the mass of lanthanum is 14-22%" can be further interpreted as "the mass of lanthanum in polystyrene sulfonic lanthanum account for 14-22% of the polystyrene sulfonate matrix". And we can give further interpretations of the mass ratio related to the mass of lanthanum in polystyrene sulfonic lanthanum as the example does.

## Claims

1. This patent relates to a medicine curing hyperphosphatemia, which is composed of polystyrene sulfonic lanthanum, the effective ingredient of the medicine, and some other pharmaceutical excipients. The following is the general molecular formula of polystyrene sulfonic lanthanum: 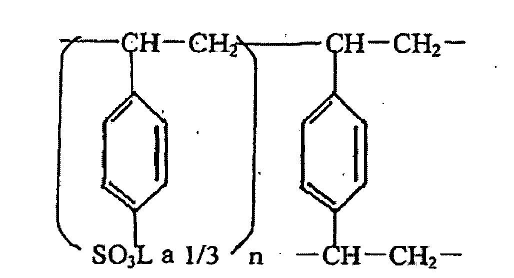 In the formula n refers to an integral number.

2. In accordance with clause 1 of the patent claim, the medicine curing hyperphosphatemia is **characterized by** its composition, in which the mass of lanthanum is generally 16~22%;

3. In accordance with clause 2 of the patent claim, the medicine curing hyperphosphatemia is **characterized by** its composition in which the mass of lanthanum is generally 16~22%;

4. The medicine curing hyperphosphatemia mentioned in clause 1, 2 or 3 of the patent claim is **characterized by** the application of polystyrene sulfonic lanthanum in treating hyperphosphatemia by gastrointestinal drug intake;

5. The medicine curing hyperphosphatemia mentioned in clause 1, 2 or 3 of the patent claim is **characterized by** its various formulation forms, including powder, capsule, tablets, dry suspension, suspension, and granules;

6. The preparation method of the medicine curing hyperphosphatemia mentioned in clause 1 , 2or 3 of the patent claim is **characterized by** the following procedures: polystyrene sulfonic salts are acidized with HCl to produce polystyrene sulfonate, which is then soaked or washed with soluble lanthanum salt solutions and rinsed to a neutral pH value with distilled water. After surplus lanthanum ions un-exchanged are removed, polystyrene sulfonic lanthanum is dried and pulverized into powder. The mass of lanthanum in polystyrene sulfonic lanthanum is 14~22%. Then in the polystyrene sulfonic lanthanum powder obtained, in which the mass of lanthanum is 14~22%, we can add conventional amounts of pharmaceutical excipients to produce medicine with conventional preparation method;

7. The preparation method, mentioned in clause 6 of the patent claim, with which the medicine curing hyperphosphatemia is produced, is **characterized by** the following procedures: the polystyrene sulfonic salts are acidized with HCl to turn into polystyrene sulfonate, which is then soaked or washed at 0~60□, and laid aside or washed for 5-30 hrs;

8. The preparation method, mentioned in clause 6 of the patent claim, with which the medicine curing hyperphosphatemia is produced, is **characterized by** the following procedures: The weight percentage of lanthanum in lanthanum polystyrene sulfonate obtained with the stated preparative method is 14%-22%;

9. The preparation method, mentioned in clause 6 , 7 or 8 of the patent claim, with which the medicine curing hyperphosphatemia is produced, is **characterized by** its various formulation forms, including powder, granules, capsules, dry suspension, suspension, and tablets;

10. The preparation method, mentioned in clause 6 , 7or 8 of the patent claim, with which the medicine curing hyperphosphatemia is produced, is **characterized by** the various soluble lanthanum solutions applied, including lanthanum nitrate solution, lanthanum chloride solution, lanthanum sulfate solution, and lanthanum acetate solution.
